# FASCICULE DE BREVET EUROPEEN

(11) **EP 1 578 714 B1**
(45) Date de publication et mention de la délivrance du brevet: **08.08.2007**
(21) Numéro de dépôt: 03799643.6
(22) Date de dépôt: 19.12.2003
(51) Int. Cl.: C07C 55/08

(54) **NOUVEAUX COMPOSES CHIRAUX DERIVES D'ESTERS DE L'ACIDE HEXANOIQUE, PROCEDE ET INTERMEDIAIRES DE PREPARATION, UTILISATION A LA SYNTHESE DE L'ACIDE 2-(BROMOMETHYL) 2-ETHYL HEXANOIQUE CHIRAL**
NEUE CHIRALE HEXANSÄUREESTERDERIVATE, VERFAHREN UND ZWISCHENPRODUKTE ZU DEREN HERSTELLUNG SOWIE DEREN VERWENDUNG ZUR HERSTELLUNG VON CHIRALER 2-(BROMMETHYL)-2-ETHYL-HEXANSÄURE
NOVEL CHIRAL COMPOUNDS DERIVED FROM HEXANOIC ACID ESTERS, METHOD AND INTERMEDIATE CHEMICALS FOR THE PREPARATION AND USE THEREOF FOR SYNTHESIS OF 2-(BROMOMETHYL)2-ETHYL HEXANOIC CHIRAL ACID

(30) Priorité: 20.12.2002 FR 0216229
(43) Date de publication de la demande: 28.09.2005
(73) Titulaire: Aventis Pharma S.A., 92160 Antony (FR)
(72) Inventeur: CROCQ-STUERGA, Véronique, F-21000 Dijon (FR); ROUSSEL, Patrick, F-94320 Thiais (FR)
(74) Mandataire: Rousseau, Pierrick Edouard
(86) Numéro de dépôt international: PCT/FR2003/003812
(87) Numéro de publication internationale: WO 2004/058678

(56) Documents cités:
- US-A- 2 250 422
- US-B1- 6 465 451
- H. S. RHINESMITH: J. AM. CHEM. SOC., vol. 58, 1936, pages 596-597, XP002268111
- ACEMOGLU ET AL.: ADVANCED SYNTHESIS & CATALYSIS, vol. 343, no. 1, 2001, page 75-77 XP002268112
- DORAN ET AL.: J. AM. CHEM. SOC, vol. 60, 1939, pages 2880-2882, XP002268113
- ARUTYUNYAN ET AL.: ARMYANSKII KHIMICHESKII ZHURNAL, vol. 30, no. 9, 1980, pages 748-754, XP009024502
- HIDEHIKO ET AL: NIPPON KAGAKU KAISHI, no. 4, 1998, pages 252-254, XP009024499
- ZALINYAN ET AL.: IZVESTIYA AKADEMII NAUK ARMYANSKOI SSR, KHIMICHESKIE NAUKI, vol. 18, no. 6, 1965, pages 600-605, XP009024489
- GILBERT ET AL.: BIOMEDICAL MASS SPECTROMETRY, vol. 1, no. 2, 1974, pages 142-144, XP009024492
- KAYAMA ET AL.: NIPPON KAGAKU KAISHI, no. 2, 1999, pages 145-148, XP009024504
- GOLDHAHN H.: PHARMAZIE, vol. 8, 1953, pages 324-327, XP009024498
- GOLDHAHN ET AL.: ARCH. PHARM. BER. DTSCH. PHARM. GES., vol. 295, no. 8, 1962, pages 598-604, XP009024458
- KOTSCHETKOW: ZH. OBSHCH. KHIM., vol. 16, 1956, pages 971-975, XP009024355
- GIGSBY ET AL: JOURNAL OF ORGANOMET. CHEM., vol. 259, no. 2, 1987, pages 171-182, XP009015038

## Description

La présente invention a pour objet des nouveaux composés chiraux dérivés d'esters de l'acide hexanoique, leurs procédé et intermédiaires de préparation et leur utilisation à la synthèse de l'acide 2-(bromomethyl)2-éthyl hexanoïque chiral.

Les documents US 2 250 422, Rhinesmith et al. (J.Am.Chem.Soc., 1936, vol.58, 596-597), Acemoglu et al. (Adv.Synth.Catal., 2001, vol.343, 75-77), Arutyunyan et al. (Armyanskii Khimicheskii Zhurnal, 1980, vol.30, 748-754), Gilbert et al. (Biomed.Mass Spectro., 1974, vol. 1, 142-144, Kayama et al. (Nippon Kagaku Kaishi, 1999, n°2, 145-148), Goldhahn et al.(Arch.Pharm.Ber.Dtsch.Pharm.Ges., 1962, vol.295,598-604), Kotschetkow (Zh.Obshch., 1956, vol.16, 971-975) et Gigsby et al. (J. of Organomet. Chem., 1987, vol. 259, 71-182) décrivent des composés qui sont des intermédiaires dans la présente demande.

L'invention a pour objet un composé chiral(R) ou (S) répondant à la formule (I) 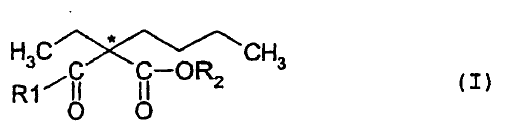 dans laquelle R₁ représente un radical hydroxy ou R'₁, R'₁ représentant un groupement activateur de la fonction acide, et R₂ représente un radical alkyle renfermant de 1 à 8 atomes de carbone, éventuellement substitué par un ou plusieurs atomes d'halogène, ou un radical benzyle.

Par radical alkyle renfermant de 1 à 8 atomes de carbone, on entend tout type d'alkyle linéaire ou ramifié, et, de préférence, un radical méthyle, éthyle ou propyle ou butyle linéaire ou ramifié.

Par atome d'halogène, on entend l'atome de fluor, de chlore, de brome ou d'iode.

Par radical alkyle substitué par halogène, on entend un radical méthyle ou, de préférence, éthyle, substitué par un ou plusieurs atomes de chlore ou de fluor. Par groupement activateur de la fonction acide, on entend n'importe quel groupement connu de l'homme du métier, par exemple, un atome de chlore ou de brome, ou un reste d'ester, par exemple dérivé de 1-hydroxybenzotriazole, de thioester, par exemple dérivé de 2-mercaptobenzothiazole, d'amide, par exemple dérivé de benzotriazole-3-oxyde, ou d'anhydride mixte, par exemple dérivé de sulfonates ou phosphates.

De tel groupements sont connus notamment dans les procédés d'acylation.

L'invention a notamment pour objet un composé de formule (I) telle que définie précédemment, dans laquelle R₁ est choisi dans le groupe constitué par un radical hydroxy, un atome de chlore ou de brome, un reste d'anhydride mixte, un reste de thioester activé, un reste d'ester activé et un reste d'amide activé, ainsi qu'un composé de formule (I) telle que définie précédemment, dans laquelle R₂ est choisi dans le groupe constitué par un radical alkyle renfermant de 1 à 4 atomes de carbone et un radical benzyle.

L'invention a également pour objet un procédé de préparation des composés de formule (I) telle que définie précédemment, caractérisé en ce que l'on traite un composé de formule (II) 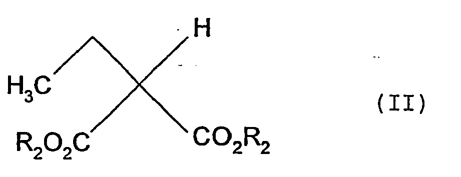 dans laquelle R₂ est défini comme précédemment, par un réactif susceptible de fixer une chaîne de formule 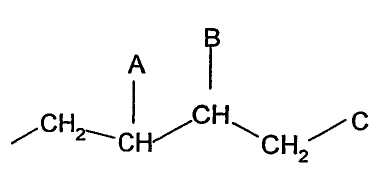 dans laquelle soit A et B représentent un atome d'hydrogène et C représente un atome de brome, soit A et B forment une deuxième liaison carbone-carbone et C représente un atome d'hydrogène, soit A et C représentent un atome d'hydrogène et
B représente une fonction cétone,
pour obtenir un composé de formule (III) 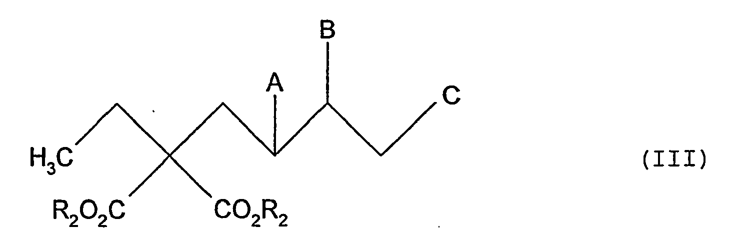 dans laquelle A, B, C et R₂ ont les significations précitées, dont, le cas échéant, on protège la fonction cétone que peut représenter B, pour obtenir un composé de formule (III') 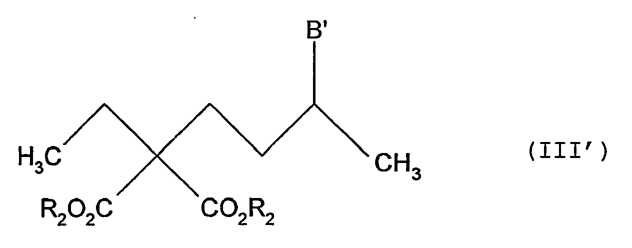 dans laquelle R₂ a la signification précitée et B' représente une fonction cétone protégée, puis traite le composé de formule (III) ou (III') par un enzyme ayant une activité hydrolytique, pour obtenir un composé chiral de formule (IV) : 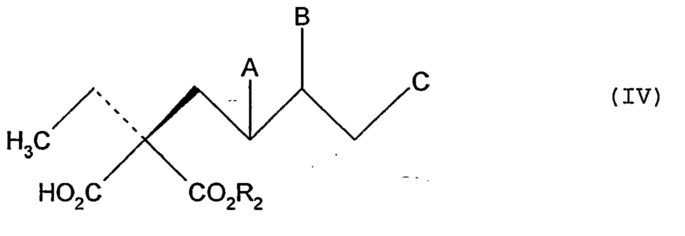 ou un composé chiral de formule (IV₁) : 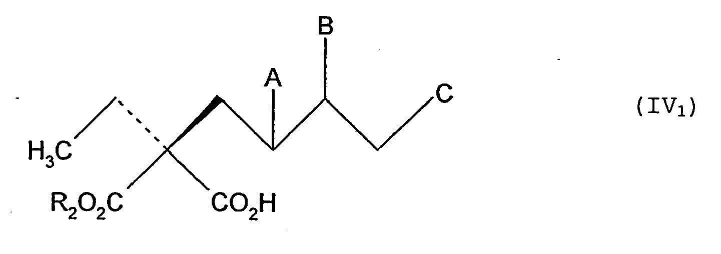 dans laquelle A, B, C et R₂ ont les significations précitées, ou un composé chiral correspondant de formule (IV') ou (IV'₁) 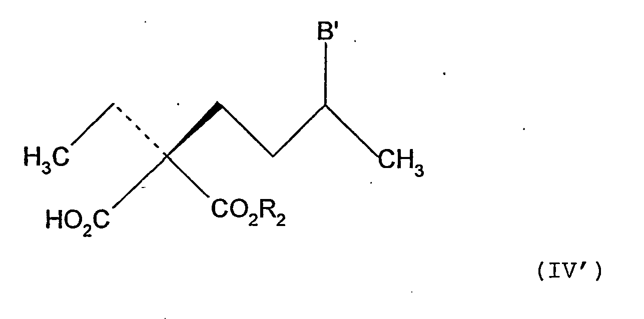 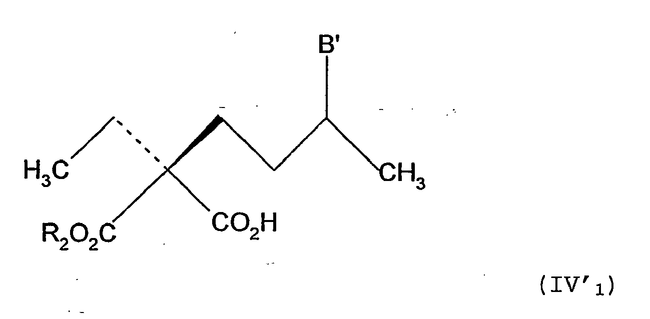 dans laquelle B' et R₂ ont les significations précitées, composé de formule (IV) ou (IV₁) ou (IV') ou (IV'₁) que l'on traite par des conditions susceptibles de générer le composé chiral correspondant de formule (I_{A}) : 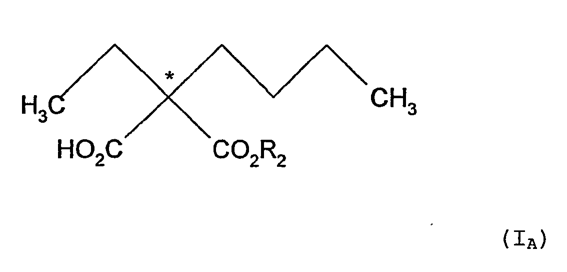 dans laquelle R₂ a la signification précitée, correspondant à un composé de formule (I) dans laquelle R₁ est un radical hydroxy, que, le cas échéant, l'on traite par un agent activateur de la fonction acide, pour obtenir un composé chiral de formule (I_{B}) 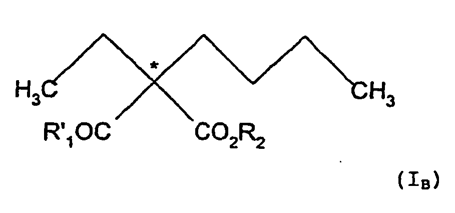 dans laquelle R₂ a la signification précitée, correspondant à un composé de formule (I) dans laquelle R'₁ a la signification précitée.

Le réactif susceptible de fixer la chaîne de formule -CH₂-CHA-CHB-CH₂C est un dérivé halogéné de ladite chaîne ou un dérivé insaturé en bout de chaîne. Des exemples figurent ci-après dans la partie expérimentale.

La protection de la fonction cétone que peut représenter B peut être toute protection connue de l'homme de métier, par exemple un cétal ou un thiocétal.

L'enzyme ayant une activité hydrolytique induisant l'asymétrie peut notamment être une estérase, une protéase ou une lipase, par exemple une estérase de-foie de porc, la chymotrypsine ou une lipase de pancréas de porc. Parmi les enzymes préférés, on peut citer notamment l'enzyme semi purifié de foie de porc connu sous le nom commercial chirazyme E₁.

Les conditions susceptibles de générer le composé chiral (I_{A}) dépendent bien entendu de la nature du composé mis en oeuvre. S'il s'agit d'un composé dans lequel A et B forment une seconde liaison ou d'un composé dans lequel C représente un atome de brome, on effectue une réduction par exemple par l'hydrogène en présence de palladium dans le tétrahydrofurane. S'il s'agit d'_un composé dans lequel B représente une fonction cétone, on effectue par exemple une réduction de type Wolff-Kishner. S'il s'agit d'un cétal, on peut effectuer une réduction par le sodium dans l'ammoniac liquide. S'il s'agit d'un thiocétal, on peut effectuer une réduction par l'hydrogène en présence d'un catalyseur métallique, notamment le nickel.

L'agent activateur de la fonction acide est un agent susceptible de former soit un chlorure ou bromure d'acide, soit un ester, soit un thioester, soit un amide, soit un anhydride mixte. De tels agents sont classiques et connus de l'homme du métier par exemple dans la mise en oeuvre de réaction d'acylation.

L'invention a encore pour objet l'application des composés de formule (I) telle que définie plus haut, à la préparation du composé chiral de formule (A): 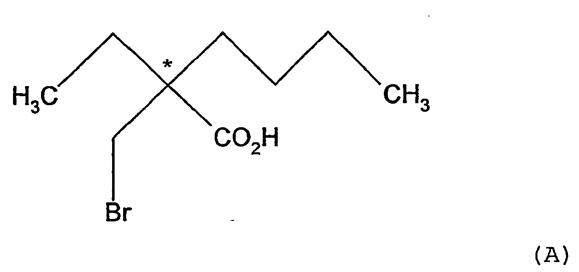 caractérisé en ce que l'on soumet un composé de formule (I) à l'action d'un agent réducteur, pour obtenir un composé chiral de formule (V) : 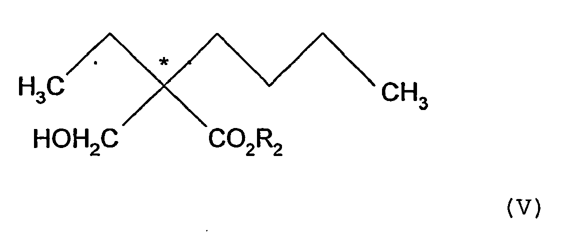 dans laquelle R₂ a la signification précitée, que l'on saponifie pour obtenir l'acide chiral de formule (VI) 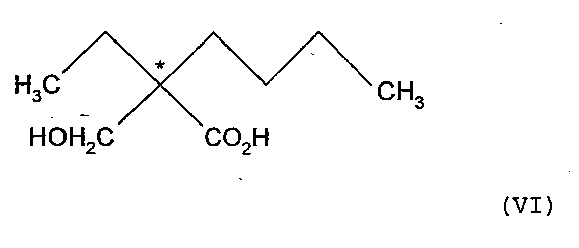 que l'on soumet à l'action d'un agent de bromation, pour obtenir le composé chiral de formule (A).

L'agent réducteur que l'on fait agir sur le composé (I) est par exemple un borohydrure alcalin tel que borohydrure de sodium. Il peut être également un alcoxyborane ou un acylborane.

Le composé (I) mis en oeuvre est de préférence un composé dans lequel R₁ représente R'₁. La saponification du composé de formule (V) peut être effectuée dans les conditions classiques connues de l'homme du métier.

L'agent de bromation est de préférence l'acide bromohydrique.Le dérivé malonate de formule (II) est connu et décrit ou préparable par des procédés décrits.

L'invention a enfin pour objet les composés de formule (III) et (III') dans lesquelles soit A et B forment une deuxième liaison carbone-carbone et C représente un atome d'hydrogène, soit A et C représentent un atome d'hydrogène et B représente une fonction cétone, et B' et R₂ sont tels que définis précédemment, ainsi que les composés chiraux de formule (IV) et (IV₁), (IV') et (IV'₁), ainsi que les composés chiraux de formule (V), (VI) et (A).

Le composé de formule (A) est utile notamment dans la synthèse de composés thérapeutiquement actifs.

Les exemples suivants illustrent l'invention sans toutefois la limiter.

### EXEMPLE 1 . Acide (2R)-2-éthyl-2-(méthoxycarbonyl)hexanoïque

### Stade A : Diméthyl 2-(4-bromobutyl)-2-éthylmalonate

On mélange sous gaz inerte 10,6 g d'éthylmalonate de diméthyle, 57,4 g de 1,4-dibromobutane et 30 cm³ de tétrahydrofurane. On refroidit à +3°C et introduit lentement 8,5 g de tert butylate de potassium dans 55 cm³ de tétrahydrofurane. Après ¼ d'heure on laisse la température remonter lentement puis maintient pendant 3 h à 25°C sous agitation. On verse dans un mélange de 150 cm³ d'eau, 50 cm³ de chlorure de méthylène et 5 cm³ d'acide chlorydrique 2N, décante et lave la phase organique à l'eau. On réextrait les phases aqueuses au chlorure de méthylène, sèche les phases organiques réunies et les concentre à sec sous pression réduite. On obtient 62 g d'une huile que l'on distille sous une pression de 1 mm Hg. On obtient 16,2 g de produit attendu.
Spectre RMN (CDCl₃): 250MHz 3.71ppm OCH3 (6H, s) ; 3.42ppm CH₂Br (2H,t),1. 9ppm CH₂ (6H,m), 1.2ppm CH₂ (2H,m), 0.8ppm CH₃ (3H,t).

### Stade B: Acide (2R)-6-bromo-2-éthyl-2-(méthoxycarbonyl) hexanoïque

On mélange 565 cm³ d'eau, 16,1 g de produit obtenu au stade A et 28,5 cm³ de diméthylsulfoxyde, chauffe à 30°C et ajuste le pH à 7 par addition de soude 1N. On ajoute 5,4 g de chirazyme E1. On agite pendant 30 h à environ 30°C en maintenant le pH à 6,75 puis filtre l'enzyme. On lave l'enzyme sur le filtre par addition progessive de 170 cm³ d'eau. On alcanilise la phase aqueuse ainsi obtenue par addition de 0,92 g de bicarbonate de sodium. On lave ensuite l'enzyme ainsi que les phases aqueuses par du chlorure de méthylène. L'ensemble des phases aqueuses réunies est acidifié à pH 2,7 par addition de 30 cm³ d'acide chlorydrique 2N. On extrait à l'ether isopropylique, lave la phase organique à l'eau, sèche, concentre à sec sous pression réduite et obtient 12,9 g de produit attendu.
Spectre RMN (CDCl₃) 250MHz 3.79 ppm OCH₃ (3H,s) ;3.40ppm CH₂Br (2H,t),2 ppm CH₂ (6H,m), 1.9 ppm CH₂ (2H,m),0.8ppm CH₃ (3H,t).
Ee=95% RMN CDCl₃ en présence de (R) Methyl Benzyl amine

### Stade C : Acide(2R)-2-éthyl-2-(méthoxycarbonyl)hexanoïque

On mélange 12,44 g de produit obtenu au stade B, 125 cm³ de tétrahydrofurane, 2,5 g de palladium à 10% sur charbon et 12,5 cm³ de triéthylamine. On place sous atmosphère d'hydrogène et maintient sous agitation vers 26, °C pendant 20 h. On filtre le catalyseur et le lave au tétrahydrofurane. On concentre le filtrat à sec sous pression réduite et le reprend par 50 cm³ d'éther isopropylique et 50 cm³ d'eau. On acidifie le mélange par addition de 15 cm³ d'acide chlorydrique 2N, décante et réextrait la phase aqueuse par 50 cm³ d'éther isopropylique. On lave les phases organiques réunies à l'eau, sèche et concentre à sec sous pression réduite. On obtient 8,91 g de produit attendu.
Spectre RMN (CDCl₃) 250MHz 3.79 ppm OCH₃ (3H,s),2 ppm CH₂ (4H,m), 1.2 ppm CH₂ (4H,m),0.8ppm CH₃ (6H,td).

### EXEMPLE 2 :(2S) méthyl-2-{[(diéthoxyphosphoryl)oxy]carbonyl}-2-éthylhexanoate

On mélange sous gaz inerte 8,5 g de produit obtenu à l'exemple 1, 42,5 cm³ de chlorure de méthylène et 8,5 cm³ de chlorophosphate de diéthyle. On introduit lentement vers 25°C, 6,3 g de 2,6-lutidine dans 8,5 cm³ de chlorure de méthylène. Après 18 h sous agitation vers 25°C, on ajoute 35 cm³ de chlorure de méthylène et 42,5 cm³ d'eau. On ajoute pendant 5 mn, décante puis lave la phase organique à l'eau. On sèche, concentre à sec sous pression réduite et obtient 16,96 g de produit attendu, que l'on conserve en solution dans 10 cm³ de chlorure de méthylène.
Spectre RMN (CDCl₃) 250MHz 4.3 ppm CH₂ (4H, q), 3.8 ppm OCH₃ (3H, s), 2 ppm CH₂ (4H, m), 1.4 ppm CH₂ + CH₃ (10H,q), 0.8ppm CH₃ (6H,t).

### EXEMPLE 3 : Acide (2R)-2-éthyl-2-(méthoxycarbonyl)hexanoïque

### Stade A : Diméthyl 2-éthyl-2-(3-oxobutyl)malonate

On mélange sous gaz inerte 10 cm³ de diméthyl 2-éthylmalonate, 20 cm³ de méthanol et 2,5 cm³ de méthyl vinyl cétone. On introduit en 1 h 7,5 cm³ de méthyl vinyl cétone et 1 cm³ de méthylate de sodium à 10% dans le méthanol. On maintient ensuite sous agitation à 26-27°C, refroidit vers 15°C puis ajoute 2 cm³ d'acide chlorydrique 1N, puis 10 cm³ d'eau. On concentre à demi volume, ajoute 90 cm³ d'eau et extrait à l'éther isopropylique. On lave la phase organique à l'eau, sèche, concentre à sec sous pression réduite et obtient 13,45 g de produit attendu.
Spectre RMN (CDCl₃) 250MHz 3.8 ppm OCH₃ (6H,s) ,2, 4ppm CH₂ (2H,t), 2.2 ppm CH₂ + CH₃ (5H, t+s), 2 ppm CH₂(4H,q),0.8ppm CH₃ (3H,t).

### Stade B : Acide (2R)-2-éthyl-2-(méthoxycarbonyl)-5-oxohexanoïque

On mélange 20 cm³ d'eau, 0,504 g de produit obtenu au stade A et 2 cm³ de diméthylsulfoxyde. On maintient sous agitation vers 33-35°C puis ajoute lentement 0,25 g de chirazyme E1 en maintenant le pH 7-7,3 par addition de soude 0,5 N. Après 2 h, on ajoute 10 cm³ de chlorure de méthylène, acidifie à pH 2 par addition de 3 cm³ d'acide chlorydrique 1N, rajoute 10 cm³ de chlorure de méthylène et décante. On lave la phase organique à l'eau, sèche et concentre à sec sous pression réduite. On obtient 0.491 g de produit attendu.
Spectre RMN (CDCl₃) 250MHz 3.8 ppm OCH₃ (3H, s), 2,4ppm CH₂ (2H, t), 2.2 ppm CH₂ + CH₃ (5H, t+s ),1.9 ppm CH₂ (4H, q), 0.8ppm CH₃ (3H,t).
Ee=96% RMN CDCl₃ en présence de (R) Methyl Benzyl amine

### Stade C : Acide(2R)-2-éthyl-2-(méthoxycarbonyl)hexanoïque

On mélange 0,25 g de produit obtenu au stade B, avec 0.23g de NH₂-NH-SO₂-C₆H₉-CH₃ et 2.5ml de DMF. On laisse agiter 2 heures, et on ajoute en 1 heure NaBH₃CN en solution dans le DMF (2ml). Après 24 h sous agitation, on isole le produit en coulant dans une solution aqueuse 10% NaHCO₃, on extrait en présence d'acétate d'éthyle pour obtenir après concentration 0.2g de produit attendu.
Spectre RMN (CDCl₃) 250MHz 3.79 ppm OCH₃ (3H,s),2 ppm CH₂ (4H,m), 1.2 ppm CH₂ (4H,m), 0.8ppm CH₃ (6H, td).

### EXEMPLE 4 : Acide (2R)-2-éthyl-2-(méthoxycarbonyl)hexanoïque

### Stade A : Diméthyl 2-éthyl-2-[2-(2-méthyl-1,3-dithiolan-2-yl)éthyl]malonate

On mélange sous gaz inerte à 20-22°C 4,65 g de produit obtenu au stade A de l'exemple 3 et 23 cm³ de toluène. On ajoute en 10 mn vers 23°C 3,7 g d'éthane dithiole et 4,25 g d'étherate de trifluorure de bore. Après 17 h sous agitation à température ambiante on verse dans un mélange de 50 cm³ d'éther isopropylique et 50 cm³ de mélange eau/glace. On agite pendant 5 mn décante, ré-extrait la phase aqueuse d'éther isopropylique, lave les phases organiques réunies à l'eau et par une solution aqueuse à 1% de bicarbonate de sodium. On sèche, concentre à sec sous pression réduite et obtient 6,64 g de produit attendu.
Spectre RMN (CDCl₃) 250MHz 3.8 ppm OCH₃ (6H,s),3,3ppm S-CH₂-CH₂-S (4H,m), 2.2 ppm CH₂-S (2H,m), 2 ppm CH₂(2H,q),1.8ppm CH₂+CH₃ (5H,m+s),0.9ppm (3H,t).

### Stade B : Acide(2R)-2-éthyl-2-(méthoxycarbonyl)-4-(2-méthyl-1,3-dithiôlan-2-yl)butanoïque

On mélange 6 cm³ d'eau, 0,124 g de produit obtenu au stade A et 0,6 cm³ de diméthylsulfoxyde. On ajoute lentement 0,125 g de chirazyme E1 en maintenant la température à 33-34°C et le pH à 7,5-8,5 par addition de soude 0,2 N. On maintient sous agitation pendant 26 h puis ajoute 6 cm³ de chlorure de méthylène et 0,6 cm³ d'acide chlorydrique 1N. On décante, ré-extrait la phase aqueuse au chlorure de méthylène, réunit les phases organiques, lave à l'eau, sèche, et concentre à sec sous pression réduite. On obtient 0,107 g de produit attendu.
Spectre RMN (CDCl₃) 250MHz 3.8 ppm OCH₃ (3H,s), 3, 3ppm S-CH₂-CH₂-S (4H, m), 2.2 ppm CH₂-S (2H, m), 2 ppm CH₂(2H,q), 1.8ppm CH₂+CH₃ (5H, m+s), 0.9ppm (3H, t).
Ee=95% RMN CDCl₃ en présence de (R) Methyl Benzyl amine

### Stade C : Acide (2R)-2-éthyl-2- (méthoxycarbonyl) hexanoïque.

On mélange 0.107 g de produit obtenu au stade B, 1 cm³ de tétrahydrofurane, 25 mg de nickel. On place sous atmosphère d'hydrogène et maintient sous agitation vers 26,°C pendant 20 h. On filtre le catalyseur et le lave au tétrahydrofurane. On concentre le filtrat à sec sous pression réduite et le reprend par 10 cm³ d'éther isopropylique et 10 cm³ d'eau. On décante et réextrait la phase aqueuse par 10 cm³ d'éther isopropylique. On lave les phases organiques réunies à l'eau, sèche et concentre à sec sous pression réduite. On obtient 0,5 g de produit attendu.
Spectre RMN (CDCl₃) 250MHz 3.79 ppm OCH₃ (3H,s),2 ppm CH₂ (4H, m), 1.2 ppm CH2 (4H, m), 0.8ppm CH3 (6H, td).

### EXEMPLE 5 : Acide (2R)-2-éthyl-2-(méthoxycarbonyl)hexanoïque

### Stade A : Diméthyl 2-éthyl-2-[2-(2-méthyl-1,3-dioxolan-2-yl)éthyl]malonate

On mélange 0,45 cm³ d'éthylène glycol, 10 mg d'acide paratoluène sulfonique et 0,88 cm³ d'orthoformiate de méthyle puis ajoute 0,92 g de produit obtenu au stade A de l'exemple 3. On maintient sous agitation pendant 24 h à température ambiante puis verse dans 20 cm³ d'une solution acqueuse à 1% de bicarbonate de sodium. On extrait au chlorure de méthylène, lave la phase organique à l'eau, sèche et concentre à sec sous pression réduite. On obtient 1,6 g de produit attendu.
Spectre RMN (CDCl₃) 250MHz 4 ppm O-CH₂-CH₂-O- (4H,s),3.75ppm OCH₃ (6H,s), 2 ppm CH₂ (4H, m),1.5 ppm CH₂(2H,)m,1.4ppm CH₃ (3H,s),0.9ppm (3H,t).

### Stade B : Acide (2R)-2-éthyl-2-(méthoxycarbonyl)-4-(2-méthyl-1,3-dioxolan-2-yl)butanoïque

On mélange 20 cm³ d'eau, 0,55 g de produit obtenu au stade A et 2 cm³ de diméthyle sulfoxide, maintient sous agitation à 30-32°C puis ajoute lentement 0,266 g de chirazyme E1 en maintenant la température vers 33°C et le pH à 7,2-7,5 par addition de soude 0,5 N. Après 24 h on ramène la température à 20°C puis ajoute 10 cm³ de chlorure de méthylène. On acidifie par addition de 2,3 cm³ d'acide chlorydrique 1N, décante, lave la phase organique à l'eau, la sèche et la concentre à sec sous pression réduite. On obtient 0,452 g de produit attendu brut.
Spectre RMN (CDCl₃) 250MHz 4 ppm O-CH₂-CH₂-O- (4H,s),3.8ppm OCH₃ (6H, s), 2 ppm CH₂(4H,m),1.6 ppm CH₂(2H,m), 1.4ppm CH₃ (3H,s),0.9ppm CH₃(3H,t).
Ee=99% RMN CDCl₃ en présence de (R) Methyl Benzyl amine

### Stade C : Acide (2R)-2-éthyl-2-(méthoxycarbonyl)hexanoïque

On mélange 0.4g du produit obtenu au stade B dans 8ml de NH₃ liquide à -70°C avec 0.22mg de Na. On maintient la température à -70°C pendant 3h. A -30°C, on ajoute en 1h une solution de NH4Cl (2ml) et on extrait à l'acétate d'éthyle. On lave la phase organique à l'eau, sèche et concentre à sec sous pression réduite. On obtient 0.31 g de produit attendu.
Spectre RMN (CDCl₃) 250MHz 3.79 ppm OCH₃ (3H,s), 2 ppm CH₂ (4H, m), 1.2 ppm CH₂ (4H,m), 0.8ppmCH₃ (6H,td).

### EXEMPLE 6 : Acide (2R)-2-éthyl-2-(méthoxycarbonyl)hexanoïque

### Stade A : (2E) diméthyl 2-[but-2-ènyl]-2-éthylmalonate

On mélange sous gaz inerte 10 cm³ de diméthyl 2-éthylmalonate, 20 cm³ de DMF et1.27g de NaH à 0°C. On introduit en 1 h 5.8g de 2 butène 4 chloro tout en maintenant la température à 0°C. Après 2h de réaction à 0°C, on introduit 10 ml de HCl 1N, puis on concentre, on ajoute 10 ml d'eau et on extrait à l'ether isopropylique. Après extrait sec, on récupère 8,2g (E/Z/85/15) d'une huile.
Spectre RMN (CDCl₃) 250MHz 4,6ppm H vilylique (H,td),4.2ppm H vinylique (H,q),3.75ppm OCH₃ (6H,s), 2,5 ppm CH₂ (2H,dd),2.4 ppm CH₃(3H,d), 1.4ppm CH₂ (2H, q), 0.9ppm (3H,t).

### Stade B : Acide (2R,4E)-2-éthyl-2-(méthoxycarbonyl)hex-4-ènoïque

On mélange 8g de produit obtenu au stade A. On ajoute lentement 8 g de chirazyme E1 en maintenant la température vers 33°C et le pH à 6,88 par addition de soude 1N. Après 24 h on extrait au chlorure de méthylène, lave la phase organique à l'eau, sèche et concentre à sec sous pression réduite. On obtient 6.8 g de produit attendu.
Spectre RMN (CDCl₃) 250MHz 4,6ppm H vinylique (H,td),4.2ppm H vinylique (H,q),3.70ppm OCH₃ (3H,s), 2,6 ppm CH₂ (2H,dd),2.5 ppm CH₃(3H,d),1.5ppm CH₂ (2H,q),0.9ppm (3H,t).
Ee=26% RMN CDCl₃ en présence de (R) Methyl Benzyl amine

### Stade C : Acide (2R)-2-éthyl-2-(méthoxycarbonyl)hexanoïque

On mélange 6 g de produit obtenu au stade B, 60 cm³ de tétrahydrofurane, 1.25 g de palladium à 10% sur charbon et 6.25 cm³ de triéthylamine. On place sous atmosphère d'hydrogène et maintient sous agitation vers 26,°C pendant 20 h. On filtre le catalyseur et le lave au tétrahydrofurane. On concentre le filtrat à sec sous pression réduite et le reprend par 25 cm³ d'éther isopropylique et 25 cm³ d'eau. On acidifie le mélange par addition de 7 cm³ d'acide chlorydrique 2N, décante et réextrait la phase aqueuse par 25 cm3 d'éther isopropylique. On lave les phases organiques réunies à l'eau, sèche et concentre à sec sous pression réduite. On obtient 5.7 g de produit attendu.
Spectre RMN (CDCl₃) 250MHz 3.79 ppm OCH₃ (3H,s),2 ppm CH₂ (4H,m), 1.2 ppm CH₂ (4H,m), 0.8ppm CH₃ (6H, td) .

### EXEMPLE 7: Acide (2S)-2-(bromométhyl)-2-éthylhexanoïque

### Stade A : (2R) méthyl-2-éthyl-2-(hydroxyméthyl)hexanoate

On concentre 27,4 g de solution d'anhydride mixte obtenue à l'exemple 2 à 16 g puis y ajoute sous agitation et sous gaz inerte 53 cm³ de diméthylformamide. On refroidit vers +2°C puis ajoute lentement 1,75 g de borohydrure de sodium. 1h45 mn après la fin de l'introduction on rajoute 0,17 g de borohydrure de sodium puis de nouveau 1 h plus tard, 0,17 g de borohydrure de sodium. On ajoute ensuite 73 cm³ d'éther isopropylique puis, à 6-10°C, en 15 mn, 35 cm³ d'une solution aqueuse à 5% d'acide tartrique. Après 5 mn sous agitation, on décante, réextrait la phase aqueuse à l'éther isopropylique puis lave les phases organiques réunies par une solution aqueuse saturée de bicarbonate de sodium puis à l'eau. On sèche, concentre à sec sous pression réduite et obtient 7,3 g de produit attendu brut que l'on chromotographie sur silice en éluant au mélange heptane-acétate d'éthyle 7-3. On obtient 7.2 g de produit purifié.
Spectre RMN (CDCl₃) 250MHz 3.79 ppm OCH₃ (3H,s),3.69 ppm CH₂OH (2H,s),2 ppm CH₂ (4H,m), 1.2 ppm CH₂ (4H,m),0.8ppm CH₃ (6H, td).

### Stade B : Acide(2R)-2-éthyl-2-(hydroxyméthyl)hexanoïque

On dissout 7g de produit obtenu au stade A dans 70ml de méthanol et on ajoute à 0°C 37 ml de soude 1N. On maintient à 0°C 1h; on concentre le milieu, on reprend par 37ml d'HCl 1N et on extrait le produit attendu avec 2*50ml d'acétate d'éthyle. On obtient 5.6g de produit attendu.
Spectre RMN (CDCl₃) 250MHz 3.69 ppm CH₂OH (2H,s),1.6 ppm CH₂ (4H,m), 1.2 ppm CH₂ (4H,m),0.8ppm CH₃ (6H,td).

### Stade C : Acide (2S)-2-(bromométhyl)-2-éthylhexanoïque

On mélange 5.6 g de produit obtenu comme décrit au stade B 34.8 cm³ d'acide bromohydrique à 62% puis porte à 92°C ± 2°C sous agitation pendant 7 heures puis laisse au repos pendant 16 heures à 20°C. On refroidit à 0°C ajoute 60 cm³ d'eau puis 9.8 cm³ de soude à 32%. On maintient sous agitation et introduit 25 cm³ de toluène puis de nouveau 50 cm³ d'eau et 50 cm³ de toluène et agite pendant 1 heure à 20°C. On décante, réextrait au toluène, réunit les phases organiques, sèche et concentre à sec sous pression réduite. On obtient 6.1 g de produit attendu brut que l'on purifie par distillation sous 2 mmHg. On obtient 3.17 g de produit attendu (Eb₂ = 118-124°C).
Spectre RMN (CDCl₃) 250MHz 3.52 ppm CH₂Br (2H, s),1.6 ppm CH₂ (4H,m), 1.2 ppm CH₂ (4H,m),0.8ppm CH₃ (6H,td). α^{D}₍₂₀₎ (1% CHCl3)=+4 °

## Revendications

1. Un composé chiral (R) ou (S)répondant à la formule (I) 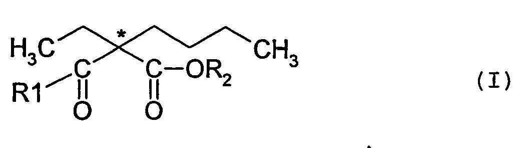 dans laquelle R₁ représente un radical hydroxy ou R'₁, R'₁ représentant un groupement activateur de la fonction acide, et R₂ représente un radical alkyle renfermant de 1 à 8 atomes de carbone, éventuellement substitué par un ou plusieurs atomes d'halogène, ou un radical benzyle.

2. Un composé de formule (I) telle que définie à la revendication 1, dans laquelle R₁ est choisi dans le groupe constitué par un radical hydroxy, un atome de chlore ou de brome, un reste d'anhydride mixte, un reste de thioester activé, un reste d'ester activé et un reste d'amide activé.

3. Un composé de formule (I) telle que définie à la revendication 1, dans laquelle R₂ est choisi dans le groupe constitué par un radical alkyle renfermant de 1 à 4 atomes de carbone et un radical benzyle.

4. Procédé de préparation des composés de formule (I) telle que définie à la revendication 1, **caractérisé en ce que** l'on traite un composé de formule (II) 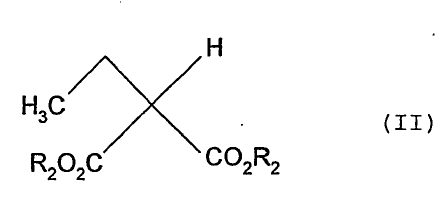 dans laquelle R₂ est défini comme à la revendication 1, par un réactif susceptible de fixer une chaîne de formule 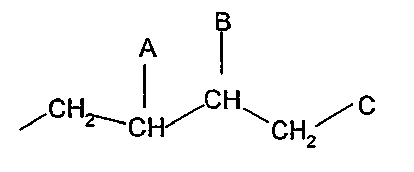 dans laquelle soit A et B représentent un atome d'hydrogène et C représente un atome de brome, soit A et B forment une deuxième liaison carbone-carbone et C représente un atome d'hydrogène, soit A et C représentent un atome d'hydrogène et B représente une fonction cétone,
pour obtenir un composé de formule (III) 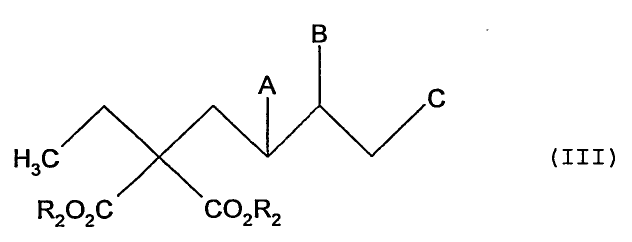 dans laquelle A, B, C et R₂ ont les significations précitées, dont, le cas échéant, on protège la fonction cétone que peut représenter B, pour obtenir un composé de formule (III') 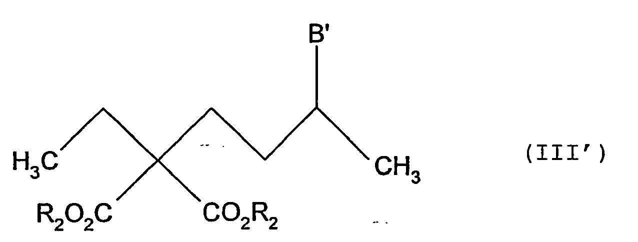 dans laquelle R₂ a la signification précitée et B' représente une fonction cétone protégée, puis traite le composé de formule (III) ou (III') par un enzyme ayant une activité hydrolytique, pour obtenir un composé chiral de formule (IV) : 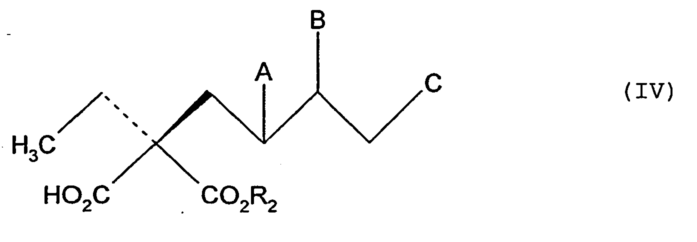 ou un composé chiral de formule (IV₁): 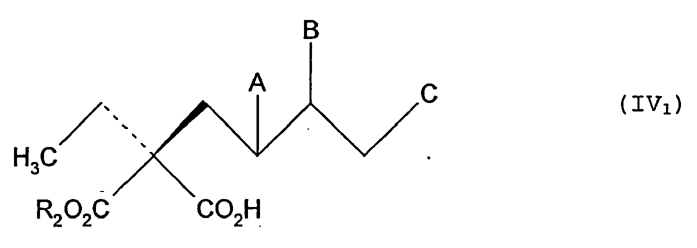 dans laquelle A, B, C et R₂ ont les significations précitées, ou un composé correspondant de formule (IV') ou (IV'₁) 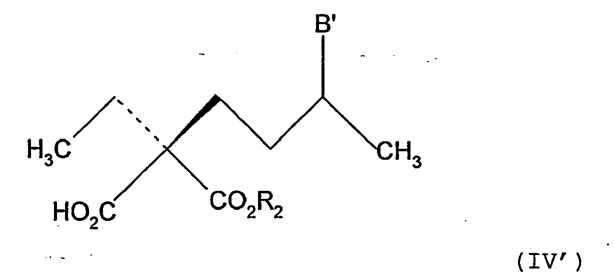 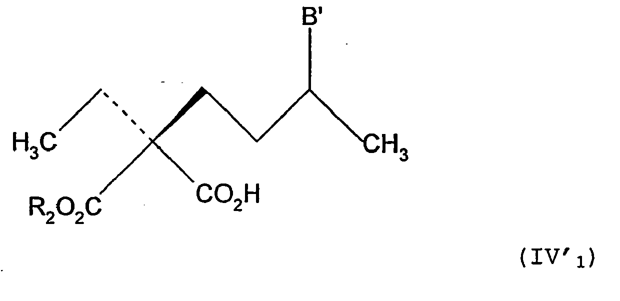 dans laquelle B' et R₂ ont les significations précitées, composé de formule (IV) ou (IV₁) ou (IV') ou (IV'₁) que l'on traite dans des conditions susceptibles de générer le composé chiral correspondant de formule (IA) : 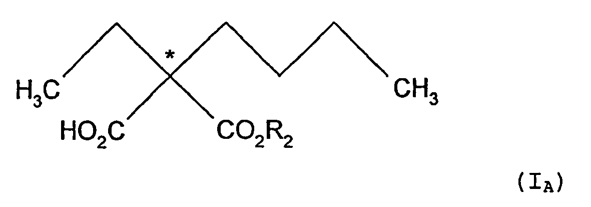 dans laquelle R₂ a la signification précitée, correspondant à un composé de formule (I) dans laquelle R₁ est un radical hydroxy, que, le cas échéant, l'on traite par un agent activateur de la fonction acide, pour obtenir un composé chiral de formule (I_{B}) 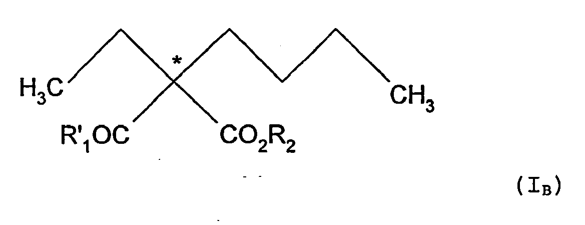 dans laquelle R₂ a la signification précitée, correspondant à un composé de formule (I) dans laquelle R'₁ est défini comme à la revendication 1.

5. Application des composés de formule (I) telle que définie à la revendication 1, à la préparation du composé chiral de formule (A) : 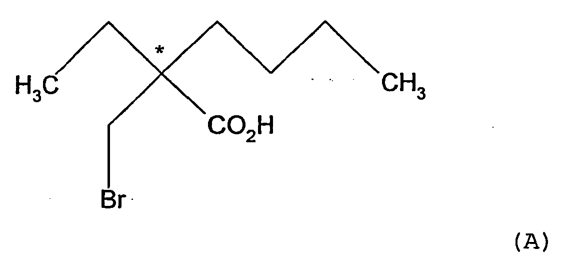 **caractérisé en ce que** l'on soumet un composé de formule (I) à l'action d'un agent réducteur, pour obtenir un composé chiral de formule (V) : 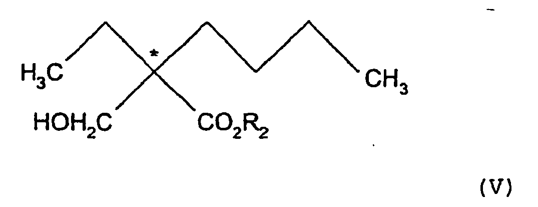 dans laquelle R₂ a la signification indiquée à la revendication 1, que l'on saponifie pour obtenir l'acide chiral de formule (VI) 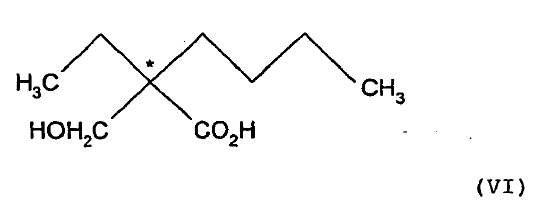 que l'on soumet à l'action d'un agent de bromation, pour obtenir le composé chiral de formule (A).

6. Application selon la revendication 5, **caractérisée en ce que** l'on met en oeuvre un composé de formule (I) dans laquelle R₁ représente R'₁, tel que défini à la revendication 1.

7. Le composé de formule: 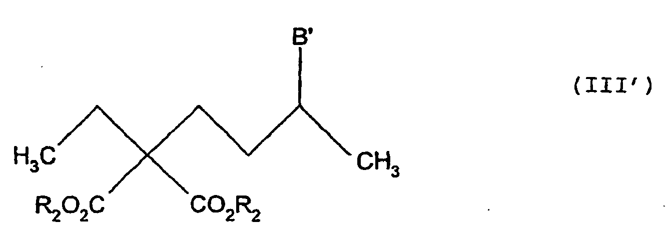 dans laquelle B' et R₂ sont tels que définis à la revendication 4.

8. Les composés chiraux de formules: 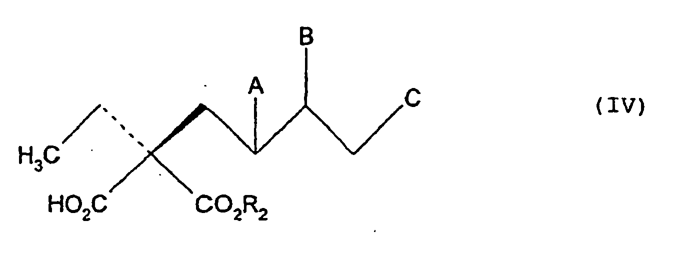 et 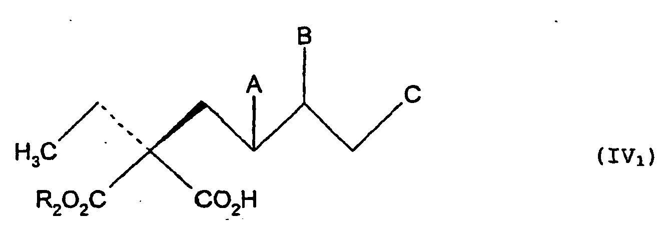 et 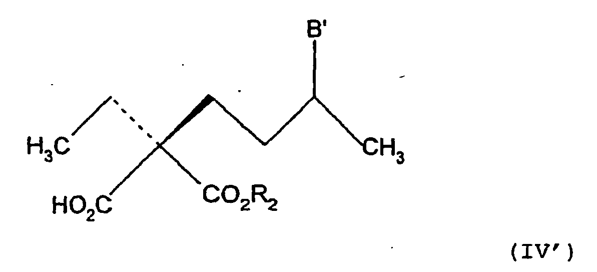 et 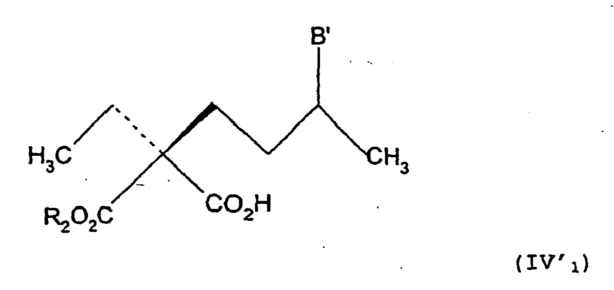 dans lesquelles A, B, C, B' et R₂ sont tels que définis à la revendication 4.

9. Les composés chiraux de formules : 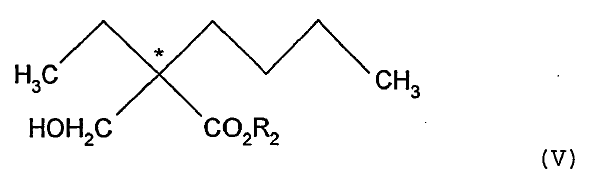 et 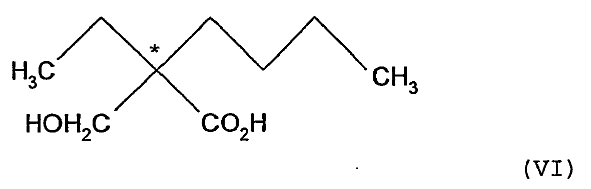 dans lesquelles R₂ est tel que défini à la revendication 5.

10. Le composé chiral de formule (A) :

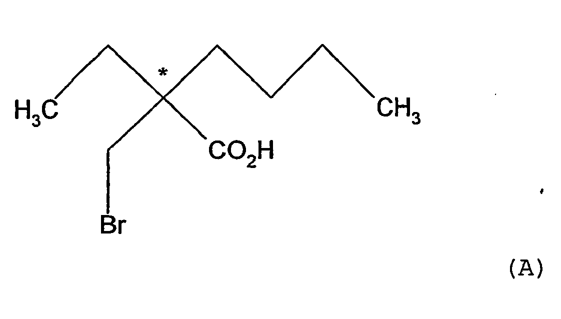

## Claims

1. (R) or (S) chiral compound corresponding to the formula (I) 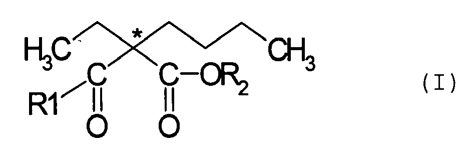 in which R₁ represents a hydroxyl radical or R'₁, R'₁ representing a group which activates the acid functional group, and R₂ represents an alkyl radical including from 1 to 8 carbon atoms, optionally substituted by one or more halogen atoms, or a benzyl radical.

2. Compound of formula (I) as defined in Claim 1, in which R₁ is chosen from the group consisting of a hydroxyl radical, a chlorine or bromine atom, a mixed anhydride residue, an activated thioester residue, an activated ester residue and an activated amide residue.

3. Compound of formula (I) as defined in Claim 1, in which R₂ is chosen from the group consisting of an alkyl radical including from 1 to 4 carbon atoms and a benzyl radical.

4. Process for the preparation of the compounds of formula (I) as defined in Claim 1, which comprises the treatment of a compound of formula (II) 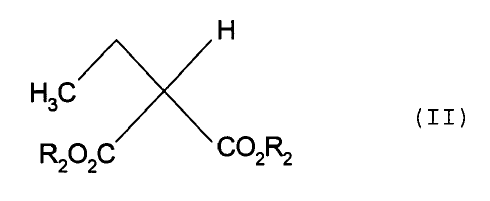 in which R₂ is defined as in Claim 1, with a reactant capable of attaching a chain of formula 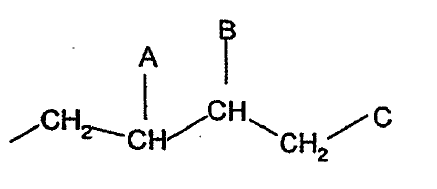 in which either A and B represent a hydrogen atom and C represents a bromine atom, or A and B form a second carbon-carbon bond and C represents a hydrogen atom, or A and C represent a hydrogen atom and B represents a ketone functional group, in order to obtain a compound of formula (III) 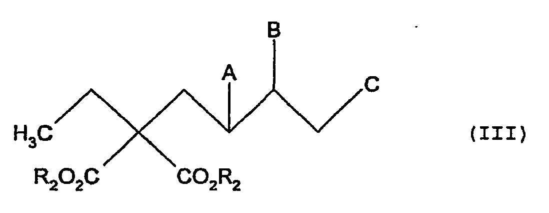 in which A, B, C and R₂ have the abovementioned meanings, the ketone functional group of which B may represent being, if appropriate, protected in order to obtain a compound of formula (III') 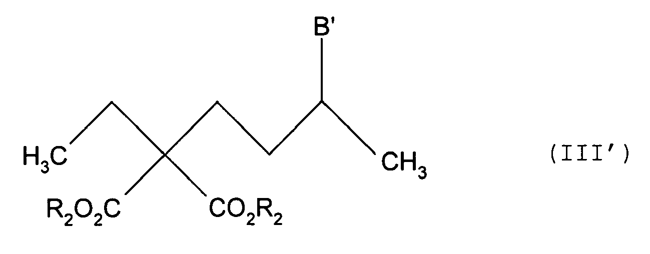 in which R₂ has the abovementioned meaning and B' represents a protected ketone functional group, then the treatment of the compound of formula (III) or (III') with an enzyme having a hydrolytic activity, in order to obtain a chiral compound of formula (IV): 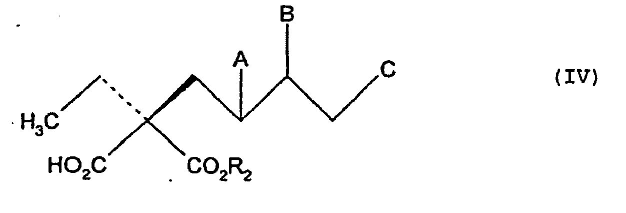 or a chiral compound of formula (IV₁): 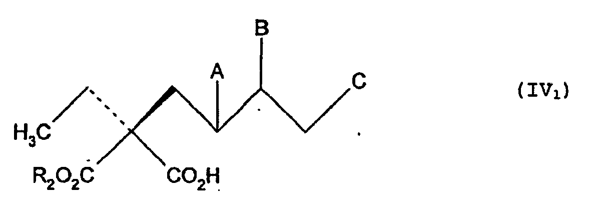 in which A, B, C and R₂ have the abovementioned meanings, or a corresponding compound of formula (IV' ) or (IV'₁) 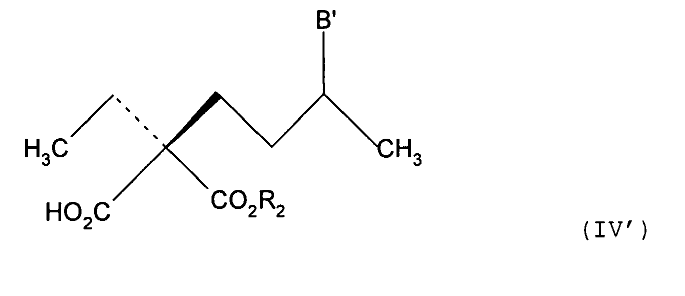 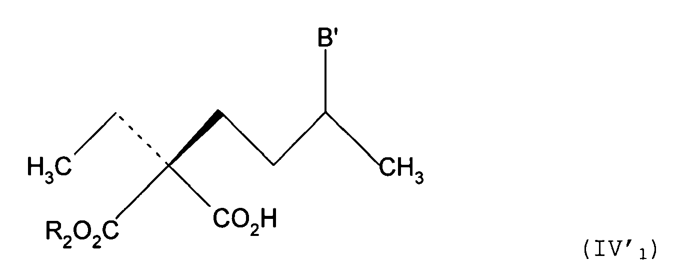 in which B' and R₂ have the abovementioned meanings, which compound of formula (IV) or (IV₁) or (IV') or (IV'₁) is treated under conditions capable of generating the corresponding chiral compound of formula (I_{A}): 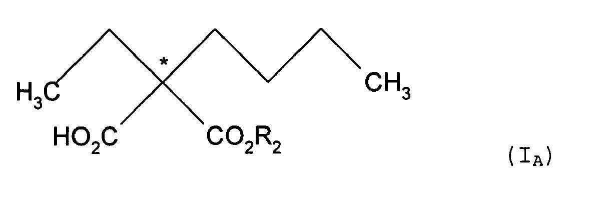 in which R₂ has the abovementioned meaning, corresponding to a compound of formula (I) in which R₁ is a hydroxyl radical, which compound, if appropriate, is treated with an agent which activates the acid functional group, in order to obtain a chiral compound of formula (I_{B}) 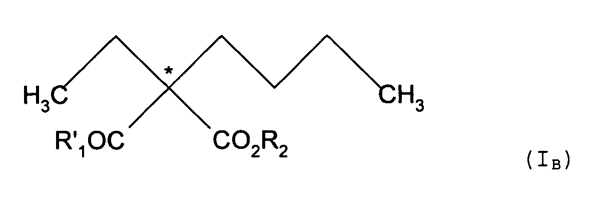 in which R₂ has the abovementioned meaning, corresponding to a compound of formula (I) in which R'₁, is defined as in Claim 1.

5. Application of the compounds of formula (I) as defined in Claim 1 in the preparation of the chiral compound of formula (A): 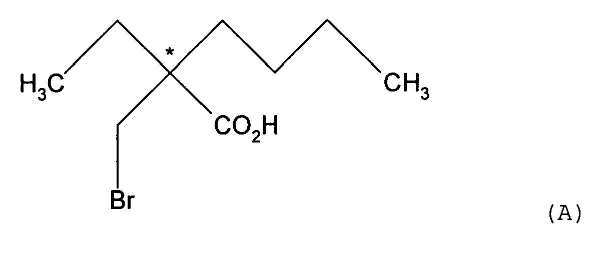 which comprises subjecting a compound of formula (I) to the action of a reducing agent in order to obtain a chiral compound of formula (V): 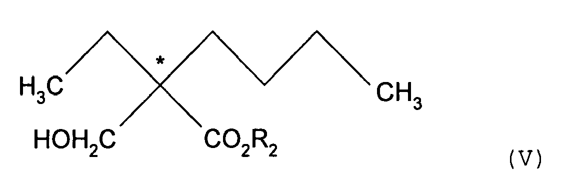 in which R₂ has the meaning indicated in Claim 1, which compound is saponified in order to obtain the chiral acid of formula (VI) 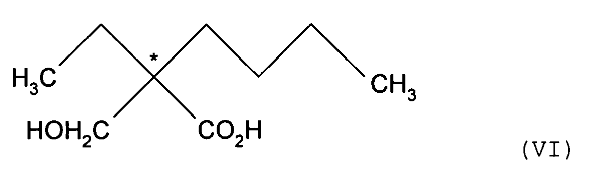 which compound is subjected to the action of a brominating agent in order to obtain the chiral compound of formula (A).

6. Application according to Claim 5, **characterized in that** use is made of a compound of formula (I) in which R₁ represents R'₁, as defined in Claim 1.

7. Compound of formula: 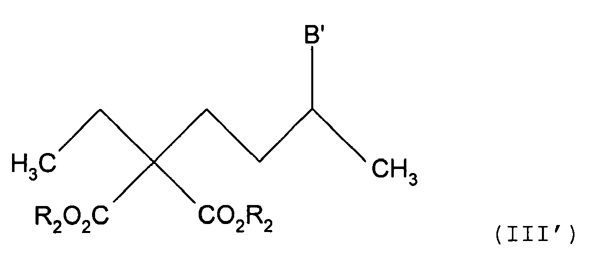 in which B' and R₂ are as defined in Claim 4.

8. Chiral compounds of formulae: 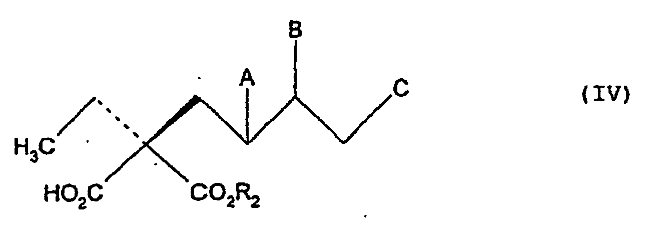 and 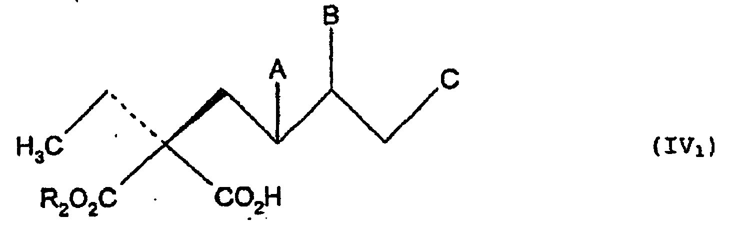 and 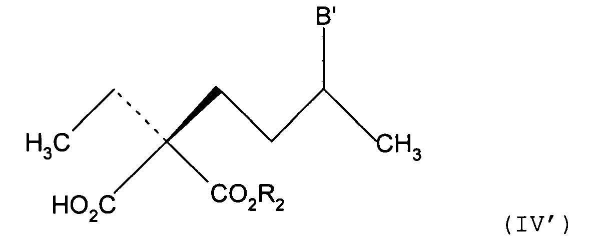 and 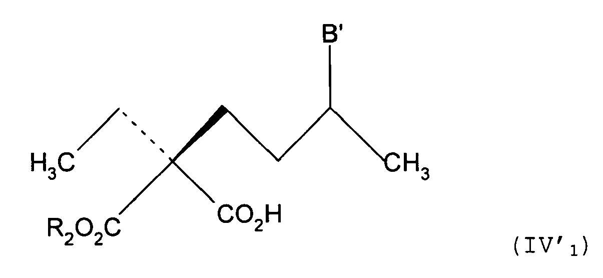 in which A, B, C, B' and R₂ are as defined in Claim 4.

9. Chiral compounds of formulae: 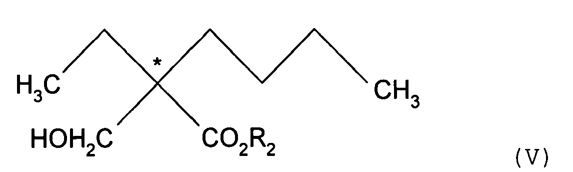 and 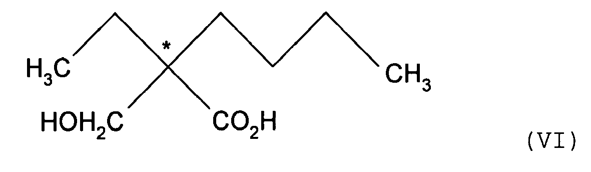 in which R₂ is as defined in Claim 5.

10. Chiral compound of formula (A):

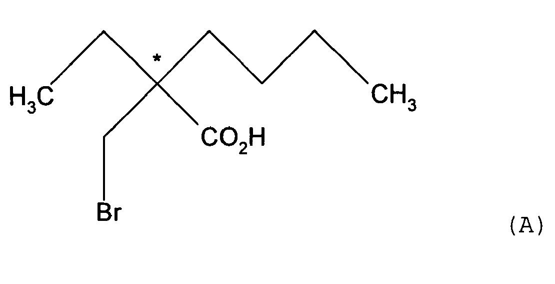

## Patentansprüche

1. (R)- oder (S)-chirale Verbindung der Formel (I), 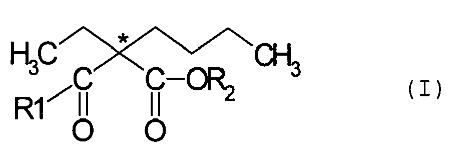 in der R₁ einen Hydroxyrest oder R'₁ bedeutet, wobei R'₁ eine säurefunktionsaktivierende Gruppe bedeutet, und R₂ einen Alkylrest mit 1 bis 8 Kohlenstoffatomen, der gegebenenfalls durch ein oder mehrere Halogenatome substituiert ist, oder einen Benzylrest bedeutet.

2. Verbindung der Formel (I) nach Anspruch 1, in der R₁ aus der Gruppe Hydroxyrest, Chlor- oder Bromatom, Mischanhydridrest, Aktivthioesterrest, Aktivesterrest und Aktivamidrest stammt.

3. Verbindung der Formel (I) nach Anspruch 1, in der R₂ aus der Gruppe Alkylrest mit 1 bis 4 Kohlenstoffatomen und Benzylrest stammt.

4. Verfahren zur Herstellung von Verbindungen der Formel (I) nach Anspruch 1, **dadurch gekennzeichnet, daß** man eine Verbindung der Formel (II), 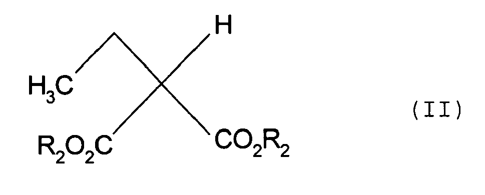 in der R₂ wie in Anspruch 1 definiert ist, mit einem Reaktanten behandelt, der eine Kette der Formel 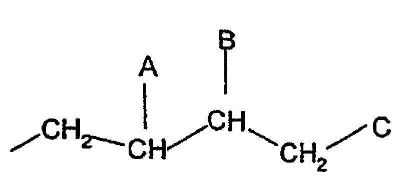 in der A und B jeweils ein Wasserstoffatom bedeuten und C ein Bromatom bedeutet oder A und B eine zweite Kohlenstoff-Kohlenstoff-Bindung bedeuten und C ein Wasserstoffatom bedeutet oder A und C ein Wasserstoffatom bedeuten und B eine Ketonfunktion bedeutet, binden kann, um zu einer Verbindung der Formel (III) 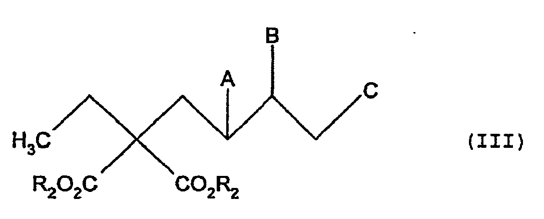 zu gelangen, in der A, B, C und R₂ die obengenannten Bedeutungen aufweisen und bei der man gegebenenfalls die Ketonfunktion, die B bedeuten kann, schützt, um zu einer Verbindung der Formel (III') 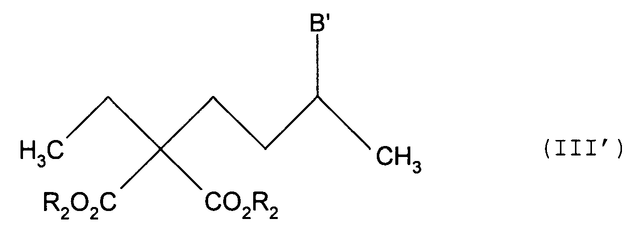 zu gelangen, in der R₂ die obengenannte Bedeutung aufweist und B' eine geschützte Ketonfunktion bedeutet, anschließend die Verbindung der Formel (III) oder (III') mit einem Enzym mit hydrolytischer Aktivität behandelt, um zu einer chiralen Verbindung der Formel (IV): 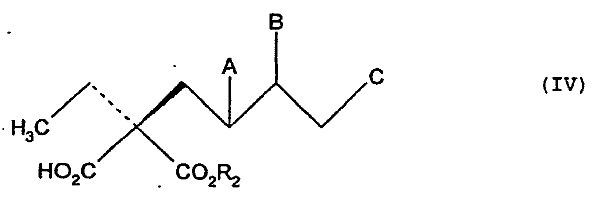 oder einer chiralen Verbindung der Formel (IV₁): 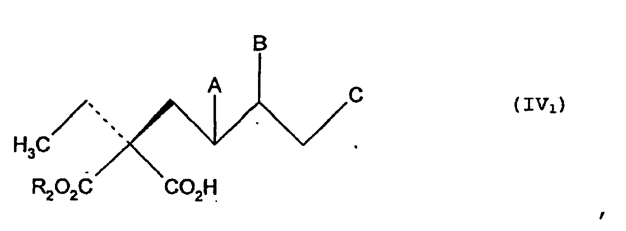 worin A, B, C und R₂ die obengenannten Bedeutungen aufweisen, oder einer entsprechenden Verbindung der Formel (IV') oder (IV'₁) 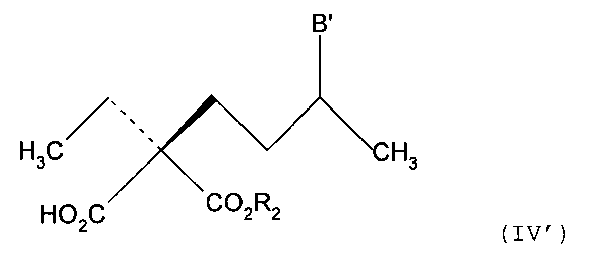 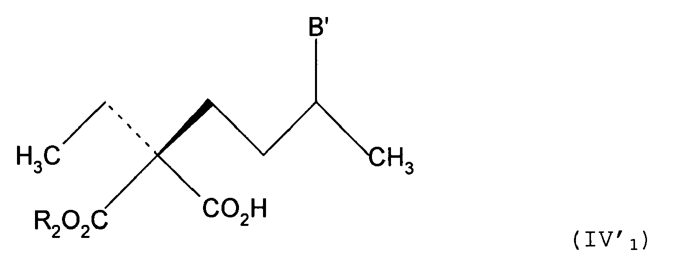 worin B' und R₂ die obengenannten Bedeutungen aufweisen, zu gelangen, und die Verbindung der Formel (IV) oder (IV₁) oder (IV') oder (IV'₁) unter Bedingungen behandelt, die die Entstehung der chiralen Verbindung der Formel (I_{A}): 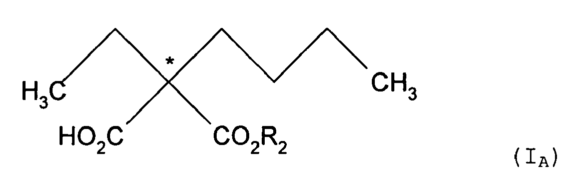 in der R₂ die obengenannte Bedeutung aufweist, ermöglichen, wobei diese Verbindung einer Verbindung der Formel (I) entspricht, in der R₁ einen Hydroxyrest bedeutet und die man gegebenenfalls mit einem säurefunktionsaktivierenden Mittel behandelt, um zu einer chiralen Verbindung der Formel (I_{B}) 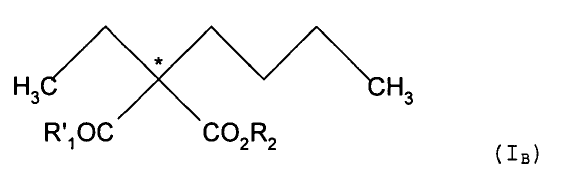 in der R₂ die obengenannte Bedeutung aufweist, zu gelangen, die einer Verbindung der Formel (I) entspricht, in der R'₁ wie in Anspruch 1 definiert ist.

5. Verwendung von Verbindungen der Formel (I) nach Anspruch 1 zur Herstellung der chiralen Verbindung der Formel (A): 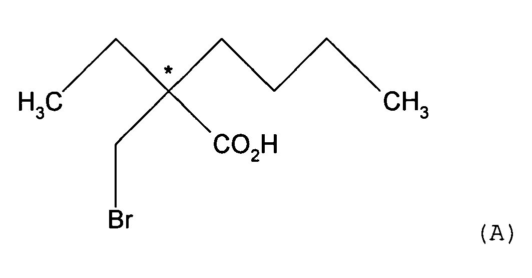 **dadurch gekennzeichnet, daß** man auf eine Verbindung der Formel (I) ein Reduktionsmittel einwirken läßt, um zu einer chiralen Verbindung der Formel (V) 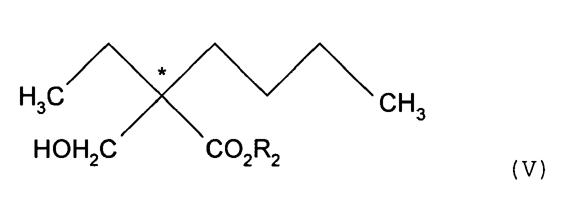 in der R₂ die in Anspruch 1 angegebene Bedeutung aufweist, zu gelangen, die man verseift, wodurch man zu der chiralen Säure der Formel (VI) 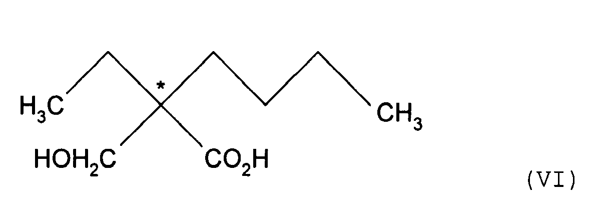 gelangt, auf die man ein Bromierungsmittel einwirken läßt, um zu der chiralen Verbindung der Formel (A) zu gelangen.

6. Verwendung nach Anspruch 5, **dadurch gekennzeichnet, daß** man eine Verbindung der Formel (I) einsetzt, in der R₁ R'₁, wie in Anspruch 1 definiert bedeutet.

7. Verbindung der Formel: 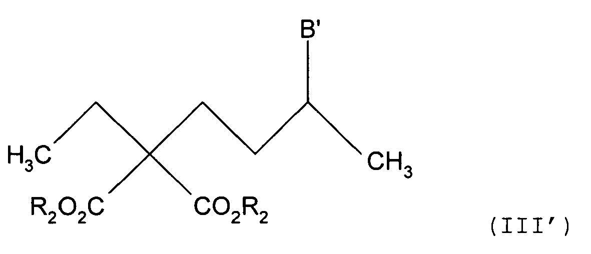 in der B' und R₂ wie in Anspruch 4 definiert sind.

8. Chirale Verbindungen der folgenden Formeln: 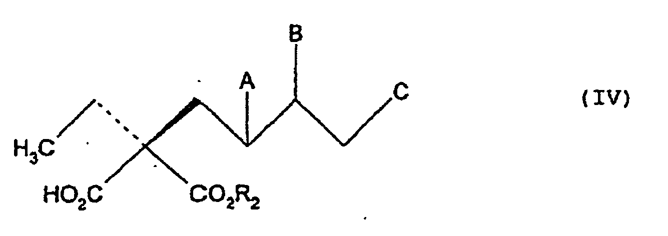 und 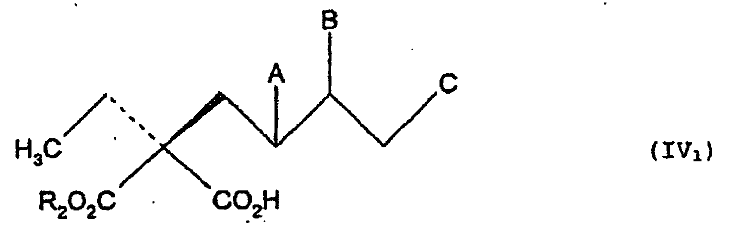 und 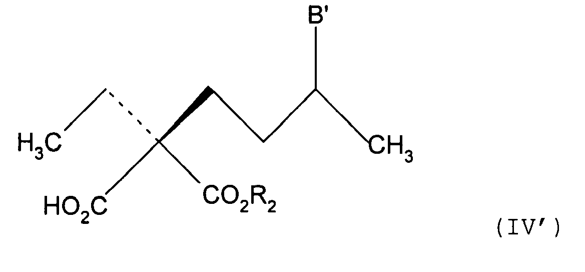 und 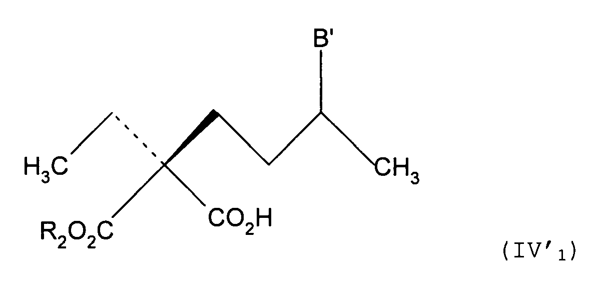 in denen A, B, C, B' und R₂ wie in Anspruch 4 definiert sind.

9. Chirale Verbindungen der folgenden Formeln: 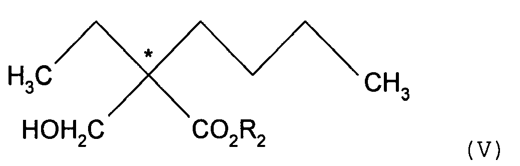 und 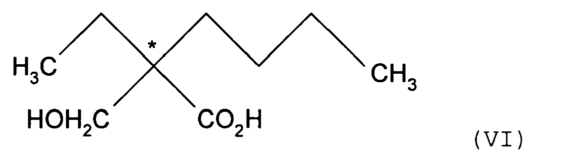 in denen R₂ wie in Anspruch 5 definiert ist.

10. Chirale Verbindung der Formel (A):
